(19) Europäisches Patentamt
European Patent Office
Office européen des brevets

(11) **EP 4 206 329 A1**

(12) **EUROPEAN PATENT APPLICATION**
published in accordance with Art. 153(4) EPC

(43) Date of publication:
**05.07.2023 Bulletin 2023/27**

(21) Application number: **21861155.6**

(22) Date of filing: **03.08.2021**

(51) International Patent Classification (IPC):
*C12P 21/08* (2006.01)     *B01J 20/04* (2006.01)
*C07K 1/14* (2006.01)      *C07K 14/005* (2006.01)
*C07K 16/00* (2006.01)     *C12N 7/02* (2006.01)

(52) Cooperative Patent Classification (CPC):
**B01J 20/04; C07K 1/14; C07K 14/005;**
**C07K 16/00; C12N 7/02**

(86) International application number:
**PCT/JP2021/028824**

(87) International publication number:
**WO 2022/044727 (03.03.2022 Gazette 2022/09)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **28.08.2020 JP 2020144757**
**28.08.2020 JP 2020144758**

(71) Applicant: **KANEKA CORPORATION**
**Osaka 530-8288 (JP)**

(72) Inventors:
• **KONOIKE, Fuminori**
  **Takasago-shi, Hyogo 676-8688 (JP)**
• **KISHI, Kenta**
  **Takasago-shi, Hyogo 676-8688 (JP)**
• **MINAKUCHI, Kazunobu**
  **Takasago-shi, Hyogo 676-8688 (JP)**
• **MURATA, Dai**
  **Takasago-shi, Hyogo 676-8688 (JP)**
• **NISHIHACHIJO, Masakatsu**
  **Takasago-shi, Hyogo 676-8688 (JP)**

(74) Representative: **Hoffmann Eitle**
**Patent- und Rechtsanwälte PartmbB**
**Arabellastraße 30**
**81925 München (DE)**

(54) **METHOD FOR PURIFYING USEFUL SUBSTANCE**

(57)     The objective of the present invention is to provide a method for purifying a useful substance with effectively suppressing the occurrence of precipitate after an impurity is adsorbed to be removed on the specific adsorbent from a useful substance-containing liquid. The method for purifying a useful substance from a useful substance-containing liquid according to the first present invention is characterized in comprising the steps of contacting the useful substance-containing liquid with a hardly-soluble magnesium compound, removing the hardly-soluble magnesium compound from the useful substance-containing liquid contacted with the hardly-soluble magnesium compound to obtain a treated liquid, and adjusting pH of the treated liquid to 8.0 or less. The method for purifying a useful substance from a useful substance-containing liquid according to the second present invention is characterized in comprising the steps of contacting the useful substance-containing liquid with a hardly-soluble magnesium compound, and contacting the useful substance-containing liquid with an aluminum compound.

EP 4 206 329 A1

**Description**

TECHNICAL FIELD

The present invention relates to a method for purifying a useful substance by using the specific adsorbent with effectively suppressing the occurrence of precipitates after impurities are adsorbed to be removed from a useful substance-containing liquid.

BACKGROUND ART

A useful peptide such as an antibody and an antibody-like molecule is generally produced using a transformed cell. In addition, a virus and a virus-like particle are also typically produced using a transformed cell and a hen egg. Such useful substances are purified from a disrupted cell suspension and a chorioallantoic fluid, and a disrupted cell suspension and a chorioallantoic fluid contain many impurities. Thus, an antibody and an antibody-like molecule are purified by affinity chromatography or the like, and a virus and a virus-like particle are purified by an ultracentrifugation method, membrane separation, chromatography or the like. Since the purification methods require high cost and a lot of trouble, it is preferred to reduce the amount of impurities preliminarily to reduce the load on the purification methods.

For example, Patent document 1 discloses the invention to purify a virus by adjusting the inorganic salt concentration and the pH of a sample liquid containing the virus to facilitate the adsorption of humic acid on the surface of hydrophobic beads. Humic acid is a mixture of polymeric organic acids that are chemically and biologically synthesized from degraded products derived from a plant residue, a microorganism and a plankton remain. It is however unclear whether a virus can be separated or not from a protein and nucleic acid by the method, since a disrupted cell suspension and a chorioallantoic fluid containing a virus also contain impurities such as a protein and nucleic acid.

Patent document 2 discloses a purification method to capture a virus-like particle on an extended bed of an adsorbent. An insoluble inorganic compound such as magnesium oxide is described as an example of the raw material for the adsorbent, but the insoluble inorganic compound is merely exemplified as an insoluble core material of the adsorbent and the actually tested adsorbent has an ion exchanger such as diethylaminoethyl (DEAE) as a ligand on the surface. Thus, the conditions such as a salt concentration and pH may be necessarily adjusted for each sample in order to selectively adsorb a target virus-like particle and not to adsorb the other protein and nucleic acid on the adsorbent.

The inventors of the present invention have developed a method for purifying an antibody or an antibody-like molecule by using basic magnesium carbonate or the like to adsorb and remove DNA or the like from a culture fluid (Patent document 3).

PRIOR ART DOCUMENT

PATENT DOCUMENT

Patent document 1: WO 2015/111606
Patent document 2: JP 2017-55766 A
Patent document 3: WO 2020/045290

DISCLOSURE OF THE INVENTION

PROBLEMS TO BE SOLVED BY THE INVENTION

The inventors of the present invention have found that basic magnesium carbonate or the like can be used for adsorbing DNA or the like and removing DNA or the like as an impurity from a culture fluid of a transformed cell to produce a useful substance as described above.

On the one hand, the inventors found the problem that precipitate is easily produced in a culture fluid when DNA or the like is adsorbed to be removed from a culture fluid by using basic magnesium carbonate or the like. For example, the inventors found the problem that after basic magnesium carbonate on which an impurity such as DNA is adsorbed is removed, precipitate is produced from the thus obtained treated liquid. The precipitate produced at this stage disturbs subsequent purification steps. For example, when a useful substance is purified by filtration and chromatography, such precipitate may cause the clogging of a filter, a column and a pipe.

The objective of the present invention is to provide a method for purifying a useful substance with effectively suppressing the occurrence of precipitate after an impurity is adsorbed to be removed on the specific adsorbent from a useful substance-containing liquid.

MEANS FOR SOLVING THE PROBLEMS

The inventors of the present invention made extensive studies to solve the above-described problem. As a result, the inventors completed the first present invention by finding that the occurrence of precipitate can be remarkably suppressed by decreasing the pH of the treated liquid after the specific adsorbent is removed.
Hereinafter, the first present invention is described.

[1] A method for purifying a useful substance from a useful substance-containing liquid, the method comprising the steps of:

contacting the useful substance-containing liquid with a hardly-soluble magnesium compound,
removing the hardly-soluble magnesium compound from the useful substance-containing liquid contacted with the hardly-soluble magnesium compound to obtain a treated liquid, and
adjusting pH of the treated liquid to 8.0 or less.

[2] The method according to the above [1], wherein pH of the useful substance-containing liquid is more than 8.0.
[3] The method according to the above [1] or [2], wherein the pH of the treated liquid is adjusted to 5.0 or more and 8.0 or less.
[4] The method according to any one of the above [1] to [3], wherein the useful substance-containing liquid is a cell culture fluid or a partially purified cell culture fluid.
[5] The method according to any one of the above [1] to [4], wherein the hardly-soluble magnesium compound is one or more selected from magnesium carbonate, magnesium hydroxide and magnesium oxide.
[6] The method according to any one of the above [1] to [5], wherein the hardly-soluble magnesium compound comprises magnesium carbonate.
[7] The method according to any one of the above [1] to [6], wherein the useful substance-containing liquid comprises nucleic acid, and the useful substance-containing liquid is contacted with the hardly-soluble magnesium compound to adsorb at least a part of the nucleic acid on the hardly-soluble magnesium compound.
[8] The method according to any one of the above [1] to [7], wherein the useful substance-containing liquid comprises an antibody or an antibody-like molecule as the useful substance.
[9] The method according to any one of the above [1] to [7], wherein the useful substance-containing liquid comprises a virus or a virus-like particle as the useful substance.
The inventors of the present invention also completed the second present invention by finding that the occurrence of precipitate can be remarkably suppressed by treating a useful substance-containing liquid with the combination of the specific adsorbents.
Hereinafter, the second present invention is described.
[10] A method for purifying a useful substance from a useful substance-containing liquid, the method comprising the steps of:

contacting the useful substance-containing liquid with a hardly-soluble magnesium compound, and
contacting the useful substance-containing liquid with an aluminum compound.

[11] The method according to the above [10], wherein the useful substance-containing liquid is contacted with the aluminum compound after the useful substance-containing liquid is contacted with the hardly-soluble magnesium compound.
[12] The method according to the above [10], wherein the useful substance-containing liquid is contacted with the hardly-soluble magnesium compound after the useful substance-containing liquid is contacted with the aluminum compound.
[13] The method according to any one of the above [10] to [12], wherein the useful substance-containing liquid is a cell culture fluid or a partially purified cell culture fluid.
[14] The method according to any one of the above [10] to [13], wherein one or more ions selected from a magnesium ion, a phosphate ion and a carbonate ion are adsorbed on the aluminum compound.
[15] The method according to any one of the above [10] to [14], wherein the hardly-soluble magnesium compound is one or more selected from magnesium carbonate, magnesium hydroxide and magnesium oxide.
[16] The method according to any one of the above [10] to [15] wherein the hardly-soluble magnesium compound comprises magnesium carbonate.
[17] The method according to any one of the above [10] to [16], wherein the aluminum compound is one or more selected from aluminum oxide and aluminum hydroxide.

[18] The method according to any one of the above [10] to [17], wherein the useful substance-containing liquid comprises an antibody or an antibody-like molecule as the useful substance.

[19] The method according to any one of the above [10] to [17], wherein the useful substance-containing liquid comprises a virus or a virus-like particle as the useful substance.

EFFECT OF THE INVENTION

The adsorbent used in the present invention method is very inexpensive and can be produced without a lot of trouble unlike the adsorbent having a ligand on the surface, since the adsorbent is the specific hardly-soluble magnesium compound or the combination of the hardly-soluble magnesium compound and an aluminum compound and can be produced without bonding an ion-exchange group and a ligand. In addition, a useful substance can be purified by the hardly-soluble magnesium compound as the adsorbent used in the present invention method, since the hardly-soluble magnesium compound can reduce a total amount of an impurity such as nucleic acid contained in a disrupted cell suspension or the like containing a useful substance such as an antibody and has the low affinity for a useful substance. The aluminum compound as another adsorbent used in the second present invention method can prevent the precipitation of precipitate in a useful substance-containing liquid, since the aluminum compound can adsorb the ion derived from the useful substance-containing liquid and the hardly-soluble magnesium compound. Such an ion constitutes precipitate. Furthermore, the precipitation of precipitate from the liquid treated by the adsorbent can be suppressed by very simple operation, i.e. decreasing pH, in the first present invention. Thus, the present invention is industrially very excellent, since a useful substance such as an antibody, an antibody-like molecule, a virus and a virus-like particle can be easily and efficiently purified by the present invention.

BRIEF DESCRIPTION OF THE DRAWINGS

Figure 1 is a graph to demonstrate the result of the analysis of the components constituting the precipitated solid in the cell culture fluid.

Figure 2 is a graph to demonstrate the result of the analysis of the ions contained in the liquid part of the untreated cell culture fluid, the liquid part of the cell culture fluid treated by basic magnesium carbonate only, and the liquid part of the treated by basic magnesium carbonate and acidic alumina.

MODE FOR CARRYING OUT THE INVENTION

Each step of the present invention method is hereinafter described, and the present invention is not restricted to the following specific examples. For example, the combination of two or more preferred embodiments of the present invention described blow is also a preferred embodiment of the present invention.

1. Production step of useful substance-containing liquid

The liquid containing a useful substance to be subjected to purification is produced in this step. This step may be carried out or may not be carried out. For example, it is not needed to carry out this step in the case where the useful substance-containing liquid is already obtained.

The useful substance is not particularly restricted as long as the useful substance is useful and is produced by a cell such as a transformed cell. An example of the useful substance includes an antibody, an antibody-like molecule, an antibody-binding protein, a virus, a virus-like particle, vaccine, a hormone, a cytokine, a growth factor, an enzyme and a plasma protein.

The term "antibody" means a protein that specifically recognizes an antigen and has the effects to bind to a foreign substance such as a pathogen inside of the body to neutralize the foreign substance, tear a hole in cell wall of bacteria to be injured and killed by activating a complement system, and promote the phagocytosis of a phagocyte. The antibody of a human is roughly classified into five isotypes of IgM, IgD, IgG, IgA and IgE, and the behaviors and the roles of each isotype are substantially different. In particular, IgG is the most abundant antibody in blood and is important for the detoxification of a risk factor and the recognition of an antigen-antibody complex by a leucocyte and a macrophage.

The term "antibody-like molecule" in this disclosure means an antibody fragment or a complex of an antibody fragment and a compound other than an antibody and an antibody fragment, such as other functional protein, and particularly means a protein having the same function as an antibody, such as a function to mediate antigen recognition and an immune reaction. The "antibody or antibody-like molecule" is not particularly restricted and is exemplified by polyclonal antibody, monoclonal antibody, human antibody, humanized antibody, chimeric antibody, single chain antibody, heavy chain antibody, polyvalent antibody, Fab, F(ab'), F(ab')$_2$, Fc, Fc-fusion protein, bispecific antibody, heavy chain (H chain), light chain (L chain), single chain Fv (scFv), sc(Fv)$_2$, disulfide-linked Fv (sdFv), diabody and antibody-like molecule target peptide (micro antibody) . The antibody or the antibody-like molecule in this disclosure may be any one of an Fc-containing protein, such as immunoglobulin and Fc-fusion protein having an Fc part, and a low-molecular antibody such as the above-described Fab, F(ab'), F(ab')$_2$, Fc, heavy chain (H chain), light chain (L chain), single chain Fv (scFv), sc(Fv)$_2$, disulfide-linked Fv (sdFv), single chain antibody, heavy chain antibody, multivalent antibody, bispecific antibody, diabody and antibody-like molecule target peptide (micro antibody).

The antibody-binding protein is not particularly restricted as long as the antibody-binding protein specifically binds to an antibody or an antibody-like molecule. An example of the antibody-binding protein includes Protein A, Protein G, Protein L, Fc$\gamma$ receptor and functional variants thereof.

The term "virus" has a structure in which DNA or RNA covered by capsid of a protein and means a complex that is not self-propagating. An example of a non-enveloped virus includes adeno-associated virus, adenovirus, enterovirus, parvovirus, papovavirus, human papillomavirus, rotavirus, coxsackievirus, sapovirus, norovirus, poliovirus, echovirus, hepatitis A virus, hepatitis E virus, rhinovirus and astrovirus. An adeno-associated virus has an AAV capsid serotype selected from the group consisting of AAV-1, AAV-2, AAV-3, AAV-4, AAV-5, AAV-6, AAV-7, AAV-8, AAV-9, AAV-10, AAV-11, AAV-12, AAV-13, AAV-14, AAV-15 and AAV-16. An example of an enveloped virus includes retrovirus, lentivirus, hemagglutinating virus of Japan, herpes simplex virus, vaccinia virus, measles virus, baculovirus and influenza virus.

The term "virus-like particle" means all of or a part of a virus outer shell protein that mainly constitutes a capsid. The virus-like particle does not raise infection concerns, since the virus-like particle does not contain nucleic acid. But the virus-like particle can be used as an active ingredient of vaccine, since the virus-like particle causes an immune reaction. In addition, the virus-like particle can be used for a drug delivery system by introducing a synthesized nucleic acid into the virus-like particle or degrading and reconstructing the virus-like particle to enclose a physiologically active substance or the like.

The term "vaccine" is a detoxified body, an attenuated body or a part structure of a pathogen such as a virus and a bacterium and means an antigen used for preventing an infective disease and the aggravation thereof by boosting immunity in a living body. For example, all of or a part of a virus outer shell protein that constitutes a capsid can be used as a virus vaccine.

The terms "hormone", "cytokine" and "growth factor" are produced in a specific cell and secreted, means a molecule to induce differentiation, motion, secretion, uptake, proliferation or the like even in a small amount by binding to a specific receptor of the other cell with a high affinity, and means a substance particularly composed of a protein in this disclosure. An example of the hormone includes insulin, glucagon, somatostatin, growth hormone, parathyroid hormone, prolactin, leptin and calcitonin. An example of the cytokine includes interleukin, interferon (IFN $\alpha$, IFN $\beta$ and IFN $\gamma$) and tumor necrosis factor (TNF). An example of the growth factor includes epidermal growth factor (EGF), insulin-like growth factor (IGF), transforming growth factor (TGF), nerve growth factor (NGF), brain-derived neurotrophic factor (BDNF), vascular endothelial growth factor (VEGF), granulocyte colony-stimulating factor (G-CSF), granulocyte-macrophage colony-stimulating factor (GM-CSF), platelet-derived growth factor (PDGF), erythropoietin (EPO), thrombopoietin (TPO), fibroblast growth factor (FGF) and hepatocellular growth factor (HGF) .

The term "enzyme" means a protein that is produced in a cell of a living body and has a catalytic activity specific to chemical change in a living body. The enzyme functions in a very small amount and is a very specific catalyst. In general, the enzyme catalyzes a specific change of a specific group of a substrate. An example of the enzyme includes lipase, protease, steroid synthetase, kinase, phosphatase, xylanase, esterase, methylase, demethylase, oxidase, reductase, cellulase, aromatase, collagenase, transglutaminase, glycosidase and chitinase.

The term "plasma protein" means a protein that is dissolved in plasma and is exemplified by thrombin, serum albumin, VII factor, VIII factor, IX factor, X factor and tissue plasminogen activator.

The useful substance-containing liquid is not particularly restricted as long as the useful substance-containing liquid is a suspension or a solution containing a useful substance in the present invention. An example of the useful substance-containing liquid includes a culture fluid of a cell that produces a useful substance and a partially purified liquid containing a useful substance obtained from a culture fluid containing a useful substance.

A cell culture fluid can be produced by cultivating a cell that produces a useful substance. For example, a host cell is transformed by using a vector containing nucleic acid encoding a useful substance to transfer the nucleic acid into the host cell to be transformed, and the transformed cell is cultivated. When a useful substance accumulates in a cell, the transformed cell is then separated from the culture fluid by centrifugation and filtration, and the separated cell is broken in a buffer solution containing a surfactant or the like. The thus obtained disrupted cell liquid may be treated by nuclease to degrade the nucleic acid derived from the host; however, an amount of expensive nuclease to be used can be reduced by the present invention method, since an impurity can be effectively removed by the present invention method. The disrupted cell liquid is centrifuged or filtrated to obtain a supernatant, and the obtained supernatant can be used as a cell culture fluid containing a useful substance. When a useful substance is discharged out of a cell, the cell may be merely removed from a culture fluid by centrifugation and filtration. In addition, a culture fluid of a wild cell that can produce a useful substance may be also used.

A publically-known cell can be used as a host cell to be transformed. An example of such a cell includes an animal cell such as HEK293 cell, CHO cell, COS cell, HeLa cell, C127 cell, 3T3 cell and BHK cell; an insect cell such as S2 cell and Sf cell; a bacterial cell such as Escherichia coli, Bacillus subtilis and bacillus bacterium; a fungal cell such as yeast and aspergillus; and a plant cell.

A useful substance-containing liquid containing a virus may be produced by an ordinary method using an egg of chicken, ostrich or the like. For example, a hen egg is sterilized and incubated 10 to 12 days, and a certain amount of a virus strain is inoculated inside of allantoic cavity to be cultivated for 2 to 3 days. Then, viral growth is arrested by cooling for about half a day. Next, the chorioallantoic fluid in which a virus grows can be used as a useful substance-containing liquid.

When a partially purified culture fluid is obtained by partially purifying a useful substance from a cell culture fluid, partial purification means is not particularly restricted as long as a useful substance can be purified by the means. An example of the means includes one or more partial purification means selected from membrane separation, chromatography, salting-out, centrifugation, demineralization, concentration and solvent exchange. The same partial purification means may be repeated 2 or more times and 5 or less times.

The concentration of a useful substance in a useful substance-containing liquid is preferably adjusted to the range that an impurity can be effectively removed. For example, when a useful substance is a protein such as an antibody, the concentration may be adjusted to 1 $\mu$g/mL or more and 1 g/mL or less. When a useful substance is a virus or a virus-like particle, the concentration may be adjusted to $10^4$ vg/mL or more and $10^{15}$ vg/mL or less.

The pH of a useful substance-containing liquid is preferably adjusted to more than 8.0 before the useful substance-containing liquid is contacted with the hardly-soluble magnesium compound in the first present invention. When the pH is more than 8.0, the hardly-soluble magnesium compound can be sufficiently prevented from being dissolved. The pH is preferably 11.0 or less. When the pH is 11.0 or less, the damage of a useful substance, such as degeneration, can be reduced more certainly. The pH is preferably 8.1 or more or 8.2 or more, more preferably 8.4 or more or 8.5 or more, even more preferably 9.0 or more, and preferably 10.5 or less or 10.0 or less, more preferably 9.5 or less.

2. Step to contact with hardly-soluble magnesium compound

At least a part of an impurity, not a useful substance, is selectively adsorbed on the hardly-soluble magnesium compound by contacting a useful substance-containing liquid with the hardly-soluble magnesium compound to increase a relative ratio of the useful substance to be purified to the impurity in the useful substance-containing liquid in this step. The term "purification" in this step means that a ratio of a useful substance to an impurity in a useful substance-containing liquid before the useful substance-containing liquid is contacted with the hardly-soluble magnesium compound is increased.

The term "hardly-solubility" means a solubility degree measured by whether dissolved or not within 30 minutes when a powdery compound to be tested is added into purified water and the mixture is vigorously shaken for 30 seconds every 5 minutes at 20±5°C in this disclosure. Specifically, the term "hardly-solubility" means that an amount of purified water required for dissolving 1 g of the compound to be tested is 400 mL or more.

The hardly-soluble magnesium compound is preferably one or more selected from magnesium carbonate, magnesium hydroxide and magnesium oxide. For example, basic magnesium carbonate, which is a mixture of magnesium hydroxide and magnesium carbonate, is also preferably used. On the one hand, a phosphate salt of magnesium is not preferred, since the phosphate salt has relatively high water-solubility and may possibly adsorb a useful substance.

The size of the hardly-soluble magnesium compound may be appropriately adjusted, and for example, the average particle diameter thereof may be adjusted to 0.1 um or more and 1000 pm or less. When the average particle diameter is 1000 pm or less, the hardly-soluble magnesium compound can more efficiently adsorb an impurity due to a sufficiently large specific surface area. When the average particle diameter is 0.1 um or more, excessive energy for pulverization is not needed. The average particle diameter of 10 um or more is preferred in terms of the handleability in the case where a column is filled with the hardly-soluble magnesium compound. The average particle diameter is measured by a laser diffraction particle size measuring device in this disclosure. An average particle diameter is based on a volume, a weight, a number or the like and is preferably based on a volume.

The shape and the structure of the hardly-soluble magnesium compound are not restricted. For example, powder, plate, needle and tube may be used. In addition, the porous hardly-soluble magnesium compound can advantageously remove an impurity due to the large area thereof. For example, tubular basic magnesium carbonate and spherical magnesium carbonate composed of assembled petal-shaped crystals (refer to JP 2008-137827 A) can be used. Such tubular basic magnesium carbonate is micro tubular particle composed of assembled leaf-like fine crystals of basic magnesium carbonate and is exemplified by "MgTube (registered trademark)" manufactured by Nittetsu Mining.

An amount of the hardly-soluble magnesium compound to be used may be adjusted depending on the concentration of a useful substance in a useful substance-containing liquid or the like, and for example, 0.1 g or more and 20 g or less of the hardly-soluble magnesium compound to 100 mL of a useful substance-containing liquid may be used. The ratio is preferably 15 g/100 mL or less. In addition, 1 mass% or more and 20 mass% or less of the hardly-soluble magnesium compound to a useful substance may be used, and the ratio is preferably 15 mass% or less.

A condition to contact a useful substance-containing liquid with the hardly-soluble magnesium compound may be appropriately selected. For example, the hardly-soluble magnesium compound is added to a useful substance-containing liquid, and the mixture may be shaken and stirred. The temperature for the contact may be ordinary temperature, and may be specifically adjusted to 1°C or higher and 30°C or lower and may be adjusted to 15°C or higher and 25°C or lower. The time for the contact may be adjusted to 5 seconds or more and 10 hours or less.

3. Step to remove hardly-soluble magnesium compound

The hardly-soluble magnesium compound is removed from the useful substance-containing liquid that has been contacted with the hardly-soluble magnesium compound in the above-described Step 2 to obtain a treated liquid in this step. This step is indispensably carried out in the first present invention and may be carried out in the second present invention.

After the contact in the above-described Step 2, the hardly-soluble magnesium compound may be separated from the treated liquid by centrifugation and filtration. A useful substance is mainly dissolved or dispersed in a liquid part and at least a part of an impurity, not a useful substance, is mainly adsorbed on the hardly-soluble magnesium compound at the time. In addition, a part of a useful substance may be possibly adsorbed on the hardly-soluble magnesium compound and a part of an impurity may be possibly dissolved or dispersed in a liquid part; however, at least a total amount of an impurity in a liquid part can be reduced and a ratio of a useful substance to an impurity is increased.

A column may be filled with the hardly-soluble magnesium compound and a useful substance-containing liquid may be passed through the column. The adsorption of an impurity and the separation of the liquid part from the hardly-soluble magnesium compound can be concurrently carried out in such a case. In other words, the above-described Step 2 and this Step 3 can be concurrently carried out. An amount of the hardly-soluble magnesium compound to fill a column and a flow velocity of a useful substance-containing liquid are preferably adjusted to the extent that an impurity is sufficiently adsorbed on the hardly-soluble magnesium compound.

A filter comprising a layer comprising the hardly-soluble magnesium compound may be prepared and a useful substance-containing liquid may be filtrated through the filter to adsorb an impurity, not a useful substance, on the filter. The adsorption of an impurity and the separation of a liquid part from the hardly-soluble magnesium compound can be also concurrently carried out in such a case. In other words, the above-described Step 2 and this Step 3 can be concurrently carried out. Such a hardly-soluble magnesium compound layer may be prepared by depositing the hardly-soluble magnesium compound on a support base material, or the hardly-soluble magnesium compound layer may be sandwiched between support base materials from the upper and the lower sides. An example of a material of a support base material layer includes one or more water-insoluble mediums selected from activated carbon; a polysaccharide such as cellulose, cellulose acetate, nitrocellulose, agarose and chitosan; a synthetic polymer such as polyacrylonitrile, polyester, polyether sulfone, polypropylene and polytetrafluoroethylene; and an inorganic substance such as diatomite, pearlite, glass, silica, alumina, zirconia and barium titanate.

An impurity to be adsorbed on the hardly-soluble magnesium compound in this step is not particularly restricted as long as the impurity is not a useful substance to be purified. An example of an impurity includes a degraded virus or a degraded virus-like particle, a contaminant derived from a host cell and a contaminant derived from a cell culture. An example of a contaminant derived from a host cell includes nucleic acid derived from a host cell, and plasmid and a protein derived from a host cell. An example of a contaminant derived from a cell culture includes a culture medium component and plasmid DNA for transfection.

4. Step to adjust pH

An impurity, not a useful substance, is selectively adsorbed on the hardly-soluble magnesium compound by contacting a useful substance-containing liquid with the hardly-soluble magnesium compound, and then the hardly-soluble magnesium compound is separated to obtain a treated liquid in the above-described Step 3. The pH of the treated liquid is adjusted to 8.0 or less in this step. This step is indispensably carried out in the first present invention. An insoluble component may be precipitated from the treated liquid and the precipitate may give damage such as clogging to a separation membrane and a column used for further purifying a useful substance in the past. Details of such an insoluble component are unknown. The hardly-soluble magnesium compound is slightly dissolved, and a magnesium ion and/or a counter anion thereof may possibly form an insoluble salt with a component contained in a culture fluid to be precipitated. On the one hand, an insoluble component is prevented from being precipitated in the present invention by lowering the pH of the treated liquid in this step. As a result, the problem caused by precipitation of an insoluble component in a purification step by membrane separation, chromatography or the like can be remarkably reduced.

An acidic pH adjuster is preferably used for adjusting the pH. An example of a pH adjuster includes an inorganic pH adjuster such as hydrochloric acid, boric acid and phosphoric acid; and an organic pH adjuster such as acetic acid, citric acid and tartaric acid. A solid pH adjuster may be used, and an aqueous solution thereof and a buffer solution thereof may be also used.

The pH of the liquid treated by the step to contact with the hardly-soluble magnesium compound is preferably lowered to 8.0 or less. When the pH value is 8.0 or less after the above-described pH adjustment, the precipitation of an insoluble component can be inhibited more surely. The pH value is preferably 5.0 or more. When the pH value is 5.0 or more, the damage of a useful substance due to low pH can be reduced more surely. The pH value is more preferably 6.0 or more, even more preferably 6.5 or more, and more preferably 7.5 or less, even more preferably 7.0 or less.

When the concentration of an ion that may form an insoluble component, especially a carbonate ion, is high, the pH of the treated liquid is preferably adjusted to be lower. For example, when the concentration of a carbonate ion in the treated liquid is 1 mol/mL or more and less than 10 mol/mL, the pH is preferably adjusted to 5 or more and 8 or less. When the concentration is more than 10 mol/mL and less than 100 mol/mL, the pH is preferably adjusted to 5 or more and 7 or less. When the concentration is 100 mol/mL or more, the pH is preferably adjusted to 5 or more and 6 or less. The precipitation of an insoluble component in the treated liquid can be suppressed more efficiently by the pH adjustment.

5. Step to contact with aluminum compound

The precipitation of an insoluble component in a useful substance-containing liquid is suppressed by contacting the useful substance-containing liquid with an aluminum compound to selectively adsorb at least a part of an ion that causes the precipitation of the insoluble component in this step. This step is indispensably carried out in the second present invention. Even when an insoluble component is removed from a useful substance-containing liquid, an insoluble component may be further precipitated and the precipitate may give damage such as clogging to a separation membrane and a column used for further purifying a useful substance in the past. Details of such an insoluble component are unknown. The hardly-soluble magnesium compound is slightly dissolved, and a magnesium ion and/or a counter anion thereof may possibly form an insoluble salt with a component contained in a culture fluid to be precipitated; a component in a useful substance-containing liquid may form an insoluble salt; and the supersaturated hardly-soluble magnesium compound may be possibly reprecipitated. On the one hand, an insoluble component is prevented from being precipitated in the present invention by removing at least a part of the ion that causes the precipitation of an insoluble component in this step. As a result, the problem caused by precipitation of an insoluble component in a purification step by membrane separation, chromatography or the like can be remarkably reduced.

The step to contact a useful substance-containing liquid with an aluminum compound may be carried out before the above-described step to contact a useful substance-containing liquid with the hardly-soluble magnesium compound, after the above-described step to contact with the hardly-soluble magnesium compound, and concurrently with the above-described step to contact with the hardly-soluble magnesium compound. When the step to contact with an aluminum compound and the above-described step to contact with the hardly-soluble magnesium compound are concurrently carried out, the hardly-soluble magnesium compound and an aluminum compound may be mixed to be contacted with a useful substance-containing liquid. The step to contact with an aluminum compound is preferably carried out after the above-described step to contact with the hardly-soluble magnesium compound. The magnesium ion that constitutes the hardly-soluble magnesium compound used in the step to contact with the hardly-soluble magnesium compound and/or the counter anion thereof may possibly form an insoluble salt, and the ions can be effectively adsorbed to be removed in this step.

The aluminum compound used in the present invention is not particularly restricted as long as the aluminum compound can adsorb the ion that causes the precipitation of an insoluble component and contains aluminum as a constituent element, and for example, is preferably one or more selected from aluminum oxide (alumina) and aluminum hydroxide. An example of aluminum oxide includes γ-alumina called as activated alumina. An activated alumina is classified into acidic alumina (pH 4), basic alumina (pH 9) and neutral alumina (pH 7.5), and acidic alumina is preferred.

The size of the aluminum compound may be appropriately adjusted, and the average particle diameter thereof may be adjusted to, for example, 0.1 μm or more and 5000 um or less. When the average particle diameter is 5000 μm or less, the specific surface area of the aluminum compound is sufficiently large and thus the aluminum compound can efficiently adsorb the constituent component of an insoluble component. When the average particle diameter is 0.1 um or more, excessive energy for pulverization is not needed. The average particle diameter is preferably 10 pm or more in terms of handleability in the case where a column is filled.

The shape and structure of the aluminum compound is not restricted, and for example, particle, clumped and spherical aluminum compounds can be used. A porous aluminum compound is advantageous to adsorption due to large specific surface area.

The usage amount of the aluminum compound may be adjusted depending of the concentration of a useful substance in a useful substance-containing liquid or the like, and for example, 0.1 g or more and 20 g or less of the aluminum compound to 100 mL of a useful substance-containing liquid may be used. The ratio is preferably 15 g/100 mL or less. In addition, 1 mass% or more and 20 mass% or less of the aluminum compound to a useful substance can be used, and the ratio is preferably 15 mass% or less.

The condition to contact a useful substance-containing liquid with the aluminum compound may be appropriately selected. For example, the aluminum compound may be added to a useful substance-containing liquid and the mixture may be shaken and stirred. The temperature during the contact may be an ordinary temperature, and may be specifically adjusted to 1°C or higher and 30°C or lower and may be adjusted to 15°C or higher and 25°C or lower. The time for the contact may be adjusted to 5 seconds or more and 10 hours or less.

The aluminum compound may be separated from a useful substance-containing liquid by centrifugation and filtration after the contact similarly to the above-described Step 4. A useful substance is mainly dissolved or dispersed in the liquid part, and at least a part of a constituent component of an insoluble component is adsorbed on the aluminum compound at the time. Apart of a useful substance may be adsorbed on the aluminum compound and a part of the constituent component of the insoluble component may be dissolved or dispersed in the liquid part in some cases but at least the total amount of the constituent component of the insoluble component in the liquid part can be reduced and the ratio of the useful substance to the constituent component of the insoluble component can be increased.

A column may be filled with the aluminum compound and a useful substance-containing liquid may be passed through the column. The adsorption of a constituent component in an insoluble component and the separation of the liquid part from the aluminum compound can be concurrently carried out in such a case. An amount of the aluminum compound to fill a column and a flow velocity of a useful substance-containing liquid are preferably adjusted to the extent that an impurity is sufficiently adsorbed on the aluminum compound. In addition, the hardly-soluble magnesium compound and the aluminum compound are mixed, and a column may be filled with the mixture. A column filled with the hardly-soluble magnesium compound and a column filled with the aluminum compound may be connected. A layer of the hardly-soluble magnesium compound and a layer of the aluminum compound may be formed in one column.

A filter comprising a layer comprising the aluminum compound may be prepared and a useful substance-containing liquid may be filtrated through the filter to adsorb a constituent component of an insoluble component on the filter. Such an aluminum compound layer may be prepared by depositing the aluminum compound on a support base material, or the aluminum compound layer may be sandwiched between support base materials from the upper and the lower sides. An example of a material of a support base material layer includes one or more water-insoluble mediums selected from activated carbon; a polysaccharide such as cellulose, cellulose acetate, nitrocellulose, agarose and chitosan; a synthetic polymer such as polyacrylonitrile, polyester, polyether sulfone, polypropylene and polytetrafluoroethylene; and an inorganic substance such as diatomite, pearlite, glass, silica, alumina, zirconia and barium titanate.

6. Step to contact with activated carbon

A useful substance-containing liquid or the treated liquid obtained by contacting a useful substance-containing liquid with the hardly-soluble magnesium compound is contacted with activated carbon to reduce the amount and the concentration of an impurity in this step. This step may be carried out or may not be carried out. The order of this step, the above-described step to contact with the hardly-soluble magnesium compound and the above-described step to contact with the aluminum compound is not particularly restricted. For example, this step may be carried out before or after the above-described step to contact with the hardly-soluble magnesium compound, the step to contact with the hardly-soluble magnesium compound and the step to contact with the aluminum compound may be carried out after the step to contact with activated carbon, and the step to contact with activated carbon may be carried out between the step to contact with the hardly-soluble magnesium compound and the step to contact with the aluminum compound. In addition, two or more steps selected from the step to contact with the hardly-soluble magnesium compound, the step to contact with the aluminum compound and the step to contact with activated carbon may be concurrently carried out by mixing two or more selected from the hardly-soluble magnesium compound, the aluminum compound and activated carbon. For example, Step 3 and this Step 6 may be concurrently carried out by mixing the hardly-soluble magnesium compound and activated carbon.

Activated carbon means a porous substance produced by burning charcoal, palm shell or the like to develop pores and is excellent in adsorption performance. A general specific surface area of activated carbon is about 800 $m^2/g$ or more and about 2500 $m^2/g$ or less. An average pore diameter of activated carbon is not particularly restricted and is generally 0.1 nm or more and 20 nm or less, preferably 0.5 nm or more and 5.0 nm or less, more preferably 2.0 nm or more and 5.0 nm or less, and even more preferably 3.0 nm or more and 5.0 nm or less. An average pore diameter of activated carbon can be calculated by BJH method on the basis of a nitrogen absorption isothermal curve. The shape of activated carbon in not particularly restricted, and an example of activated carbon includes granular activated carbon such as pulverized charcoal, granular charcoal, spherical charcoal and pellet charcoal; fibrous activated carbon such as fiber and cloth; specially formed activated carbon such as sheet, compact and honeycomb; and activated carbon powder.

A purification means using activated carbon is not particularly restricted in the present invention and is exemplified by a batch method, a membrane treatment method and a column chromatography method. The preferred shape of activated carbon may be selected depending on each means . Activated carbon can be also used as needed in a particle shape prepared by enclosing activated carbon in a porous polymer or a gel, a membrane prepared by using a supporting agent or a fiber such as polypropylene and cellulose to adsorb, immobilize or shape activated carbon, or a cartridge form.

The usage amount of activated carbon may be adjusted depending on a concentration of a useful substance in a useful substance-containing liquid or the treated liquid, and for example, 0.5 g or more and 5 g or less of activated carbon may be used to 100 mL of a useful substance-containing liquid or the treated liquid.

Activated carbon may be added to a useful substance-containing liquid or the treated liquid and the mixture may be shaken or stirred, and then the activated carbon may be separated by centrifugation or filtration, or a column may be filled with activated carbon and a useful substance-containing liquid or the treated liquid may be passed through the column similarly to the cases of the hardly-soluble magnesium compound and the aluminum compound with respect to a condition to contact a useful substance-containing liquid or the treated liquid with activated carbon. When this Step and the Steps to contact with the hardly-soluble magnesium compound and/or the aluminum compound are concurrently carried out, the hardly-soluble magnesium compound and/or the aluminum compound and activated carbon may be mixed and the mixture may be used.

7. Further purification step

When a useful substance cannot be sufficiently purified by one of the above-described step to contact with the hardly-soluble magnesium compound, the step to contact with the aluminum compound, the step to contact with activated carbon and the combination of two or more of the steps one time, the steps and the combination may be repeated two or more times. The upper limit of the repeat number is not particularly restricted, and the repeat number may be 10 times or less and is preferably 5 times or less. Since a useful substance may not be generally purified to be isolated by only the step to contact with the hardly-soluble magnesium compound, the step to contact with the aluminum compound or the step to contact with activated carbon, a publicly-known purification method is preferably carried out after the step to contact with the hardly-soluble magnesium compound, the optional step to contact with the aluminum compound or the combination of the steps and the step to contact with activated carbon. The inhibition of the purification caused by the precipitation of an insoluble component is suppressed in such a case, since the precipitation of an insoluble component is suppressed by the above-described step to adjust pH or the step to contact with the aluminum compound. In addition, a load on purification step can be remarkably reduced, since the concentration of an impurity is sufficiently reduced and thus the target useful substance is concentrated by the step to contact with the hardly-soluble magnesium compound according to the present invention. An example of a publicly-known purification method includes ultracentrifugation, membrane separation and chromatography, and membrane separation and chromatography are preferred in terms of mass production. The nucleic acid that contained in the treated liquid and is derived from a host may be treated using nuclease to degrade the nucleic acid after the step to contact with the hardly-soluble magnesium compound. The usage amount of expensive nuclease can be reduced by the present invention method, since an impurity can be effectively removed by the present invention method.

An example of chromatography includes affinity chromatography and ion exchange chromatography, and affinity chromatography is particularly preferred. Purification is efficiently possible by affinity chromatography, since the pH adjustment and the adjustment of salt concentration in a liquid in advance are not needed.

In general, when a virus or a virus-like particle is purified by chromatography, an impurity is removed by ultrafiltration and a virus or a virus-like particle is concentrated in advance, since the amount of a virus in a useful substance-containing liquid is small and an amount of an impurity is large in the case where a useful substance to be purified is a virus. On the one hand, purification by chromatography without previous ultrafiltration is possible after a useful substance-containing liquid containing a virus or a virus-like particle is treated with the hardly-soluble magnesium compound by the present invention.

A useful substance may be concentrated by reducing a solvent amount and a solvent may be exchanged after a useful substance is purified by this step. A solution or a suspension of a useful substance having higher purity and an isolated and purified useful substance can be efficiently produced by the above-described method for purifying a useful substance according to the present invention.

The present application claims the benefit of the priority dates of Japanese patent application No. 2020-144757 and Japanese patent application No. 2020-144758 filed on August 28, 2020. All of the contents of the Japanese patent application No. 2020-144757 and Japanese patent application No. 2020-144758 filed on August 28, 2020, are incorporated by reference herein.

EXAMPLES

Hereinafter, the present invention is described in more detail with Examples. The present invention is however not restricted to the following Examples in any way, and it is possible to work the present invention according to the Examples with an additional appropriate change within the range of the above descriptions and the following descriptions. Such a changed embodiment is also included in the technical scope of the present invention.

Example 1: Example of the first present invention

A resin column having an inner diameter of 0.7 cm and a length of 2.5 cm manufactured by Tomoe Works was filled with basic magnesium carbonate (1 g) and connected to a chromatography system ("AKTA Avant 25" manufactured by GE Healthcare). A syringe pump ("YSP-101" manufactured by YMC) was connected to the outlet of the column, and a static mixer was installed downstream thereof.

A CHO culture fluid containing 3.5 mg/mL of IgG (100 mL, pH 8.18) was supplied to the column at a rate of 1 mL/min, and concurrently 1 M citrate buffer (pH 5.5) was supplied at a rate of 0.08 mL/min using a syringe pump. The treated liquid was obtained in increments of 10 mL in 15 mL centrifuge tubes. The total amount of the obtained treated liquid was 100 mL. The pH of the treated liquid was lowered to 7.19 by adding the citrate buffer.

Each of the treated liquid was filtrated through a filter (pore diameter: 1 μm) manufactured by Kiriyama Glass Works, and the weight of the precipitate obtained by filtration was measured.

In addition, the IgG contained in the filtrate was captured by Protein A affinity column ("TSKgel Protein A-5PW" manufactured by Tosoh), and the IgG concentration in the obtained eluate was calculated. The HCP concentration in the obtained eluate was calculated using a kit for detecting a host cell-derived protein (HCP) ("CHO Host Cell Protein ELISA Kit, 3rd Generation" manufactured by Cygnus) . In addition, the DNA concentration in the obtained eluate was calculated using a kit for detecting a host cell-derived DNA ("CHO DNA Amplification Kit in Tubes" manufactured by Cygnus). The result is shown in Table 1.

Comparative example 1

The eluate was obtained similarly to Example 1 except that the citrate buffer was not supplied, and the concentrations of IgG, HCP and DNA were calculated. The result is shown in Table 1. The pH of the eluate was increased to 8.60 presumably due to basic magnesium carbonate.

Table 1

|  | pH | Solid precipitation amount | IgG concentration | HCP concentration | DNA concentration |
|---|---|---|---|---|---|
| Culture fluid | 8.18 | - | 3.51 g/L | 175 μg/mL | 0.574 ng/mL |
| Comparative example 1 | 8.60 | 42 mg | 3.45 g/L | 168 μg/mL | 0.019 ng/mL |
| Example 1 | 7.19 | 0 mg | 3.46 g/L* | 150 μg/mL* | 0.003 ng/mL* |

The values (*) of the IgG concentration, the HCP concentration and the DNA concentration of Example 1 in Table 1 correspond to the value calculated by multiplying the calculated value by 1.08 for the comparison with the results of the culture fluid and Comparative example 1, since the liquid treated by the column was diluted by 8% using the citrate buffer.

A solid was precipitated in the treated liquid in the case where the culture fluid was treated with basic magnesium carbonate only in Comparative example 1 as the result shown in Table 1. The solid may have a bad effect on the subsequent chromatography and filtration to purify IgG.

On the one hand, when the pH of the treated liquid obtained by treating the culture fluid with basic magnesium carbonate was adjusted to 7.19 by adding the acidic buffer, a solid was not precipitated and IgG could be obtained while the concentrations of HCP and DNA could be effectively reduced.

Example 2 - Example of the first present invention

Basic magnesium carbonate was added to 0.1 g/L salmon sperm-derived DNA-containing solution, IgG-containing CHO culture supernatant, culture medium for HEK cell ("Advanced DMEM" manufactured by gibco) or culture medium for CHO cell ("ExpiCHO Expression Medium" manufactured by gibco) prepared using each buffer in a concentration of 1 mass%, and the mixture was stirred. Phosphate buffered saline (PBS), 25 mM MES (pH 7.0), 25 mM HEPES (pH 7.0), 25 mM tricine (pH 7.8) and 25 mM tris buffer (pH 7.5) were used for preparing the salmon sperm-derived DNA-containing solution. Then, the mixture was centrifuged at 15,000 rpm for 5 minutes to spin down basic magnesium carbonate and obtain the supernatant.

The thus obtained supernatant was neutralized by adding 1 M citrate buffer (pH 5.5).

The absorbance at UV 260 nm of the neutralized supernatant was measured to obtain the DNA concentration.

Next, it was confirmed whether precipitate was absent or present after the neutralized supernatant was once frozen and then melted. The result is shown in Table 2.

Comparative example 2

The supernatant was obtained similarly to Example 2 except that the reutilization using the citrate buffer was not carried out, and the concentration of DNA therein was measured. The DNA removal rate was calculated in accordance with the following formula, and it was confirmed whether a solid was precipitated or not. The result is shown in Table 2.

```
DNA removal rate = [1 - (DNA concentration after treatment) / (DNA
concentration before treatment)] × 100
```

Table 2

| | | pH | DNA removal rate | Solid precipitation |
|---|---|---|---|---|
| Salmon-derived DNA solution (in PBS) | Untreated | 7.5 | 0% | Absent |
| | Comparative example 2 | 9.4 | 59% | Present |
| | Example 2 | 7.0 | 59% | Absent |
| Salmon-derived DNA solution (in MES buffer) | Untreated | 7.5 | 0% | Absent |
| | Comparative example 2 | 9.5 | 73% | Present |
| | Example 2 | 7.1 | 72% | Absent |
| Salmon-derived DNA solution (in HEPES buffer) | Untreated | 7.5 | 0% | Absent |
| | Comparative example 2 | 8.6 | 82% | Present |
| | Example 2 | 7.0 | 810 | Absent |
| Salmon-derived DNA solution in tricine buffer) | Untreated | 7.5 | 0% | Absent |
| | Comparative example 2 | 8.8 | 77% | Present |
| | Example 2 | 7.0 | 77% | Absent |
| Salmon-derived DNA solution (in Tris buffer) | Untreated | 7.5 | 0% | Absent |
| | Comparative example 2 | 8.7 | 79% | Present |
| | Example 2 | 7.1 | 78% | Absent |
| CHO culture supernatant | Untreated | 7.5 | 0% | Absent |
| | Comparative example 2 | 8.4 | 99% | Present |
| | Example 2 | 7.0 | 99% | Absent |

(continued)

|  |  | pH | DNA removal rate | Solid precipitation |
|---|---|---|---|---|
| Culture medium for CHO | Untreated | 7.6 | 0% | Absent |
| | Comparative example 2 | 8.4 | 99% | Present |
| | Example 2 | 7.1 | 98% | Absent |
| Culture medium for HEK | Untreated | 8.2 | 0% | Absent |
| | Comparative example 2 | 8.7 | 93% | Present |
| | Example 2 | 7.1 | 90% | Absent |

The pH of the solution containing DNA as an impurity was relatively high and thus a solid was precipitated after the solution was once frozen and then melted as the result shown in Table 2. The slight amount of the basic magnesium carbonate used for adsorbing DNA may be dissolved in the solution and each of the ion component thereof may form the insoluble salts to be precipitated as the solid.

On the one hand, when basic magnesium carbonate was removed and then the solution was neutralized, DNA was removed similarly to the case where the solution was not neutralized and a solid was not precipitated in the supernatant. Thus, it was demonstrated that basic magnesium carbonate can be effectively prevented from being precipitated by adjusting pH after the treatment using basic magnesium carbonate according to the present invention.

Example 3: Suppression of insoluble solid precipitation after culture fluid was treated with hardly-soluble magnesium compound - example of the second present invention

(1) Adsorption treatment

Basic magnesium carbonate manufactured by KISHIDA CHEMICAL was added to IgG-containing CHO culture fluid in a concentration of 1 w/v%, and the mixture was stirred using a mix rotor under room temperature for 1 hour. Then, the mixture was centrifuged at 5,000 rpm for 1 minute to obtain a supernatant as the first treated liquid. Each of the second adsorbent described in Table 1 was added to the first treated liquid in a concentration of 1 w/v%, and the mixture was stirred using a mix rotor under room temperature for 1 hour. Then, the mixture was centrifuged at 5, 000 rpm for 1 minute to obtain a supernatant as the second treated liquid.

(2) Measurement of precipitate amount

The above-described second treated liquid was frozen at -40°C and then melted to accelerate the precipitation. The treated liquid was cooled for 3 days or more, and the precipitated solid in the treated liquid was obtained by filtration and the weight thereof was measured. The result is shown in Table 3.

Table 3

| | 1st Adsorbent | 2nd Adsorbent | Solid precipitation amount |
|---|---|---|---|
| Comparative example 3 | - | - | 0 mg/L |
| Comparative example 4 | Basic magnesium carbonate | - | 389 mg/L |
| Comparative example 5 | | Diatomite | 344 mg/L |
| Comparative example 6 | | Neutral silica | 311 mg/L |
| Comparative example 7 | | Amine silica | 344 mg/L |
| Comparative example 8 | | Carbonate silica | 253 mg/L |
| Comparative example 9 | | Sulfonate silica | 281 mg/L |
| Example 3 | | Acidic alumina | 0 mg/L |

When the untreated liquid of Comparative example 3 was treated with basic magnesium carbonate, 389 mg of a solid was precipitated per 1 L of the culture fluid (Comparative example 4) as the result shown in Table 3. Even if the liquid of Comparative example 4 was treated with diatomite manufactured by FUJIFILM Wako Pure Chemical and various silica gels manufactured by FUJI SILYSIA CHEMICAL as the second treatment solid, the precipitation was not significantly suppressed as Comparative examples 5 to 9. Diatomite has been widely used as a filter aid, and various silica gels have been widely used for purification by adsorption. On the one hand, when acidic alumina manufactured by Merck was used as the second treatment solid, the precipitation was significantly suppressed as Example 3.

(3) Measurement of IgG recovery rate

The above-described second treated liquid was analyzed using a general chromatography system having a Protein A affinity column ("TSKgel Protein A-5PW" manufactured by Tosoh) to measure the amount of IgG in the liquid, and then the ratio to the initial IgG amount contained in the IgG-containing CHO culture fluid was calculated. The result is shown in Table 4.

(4) Measurement of impurity amount

The amount of host cell protein (HCP) in the above-described second treated liquid was measured using a kit for detecting host cell protein ("CHO Host Cell Protein ELISA Kit, 3rd Generation" manufactured by Cygnus) in accordance with the accompanying protocol. The result is shown in Table 4.

The amount of DNA in the above-described second treated liquid was measured using a kit for detecting the DNA derived from host cell ("CHO DNA Amplification Kit in Tubes" manufactured by Cygnus) in accordance with the accompanying protocol. The result is shown in Table 4.

Table 4

|  | 1st Adsorbent | 2nd Adsorbent | IgG recovery rate | HCP concentration | DNA concentration |
|---|---|---|---|---|---|
| Comparative example 3 | - | - | 100% | 191 $\mu$g/mL | 510 pg/mL |
| Comparative example 4 | Basic magnesium carbonate | - | 99% | 176 $\mu$g/mL | 13 pg/mL |
| Example 3 | | Acidic alumina | 99% | 167 $\mu$g/mL | 5 pg/mL |

It was found as the result shown in Table 4 that the recovery rate of the target IgG is not decreased and the amounts of HCP and DNA can be reduced, even when the treatment to suppress precipitation by acidic alumina is further carried out (Example 3) in addition to the treatment to remove an impurity using basic magnesium carbonate (Comparative example 4).

(5) Analysis of precipitate

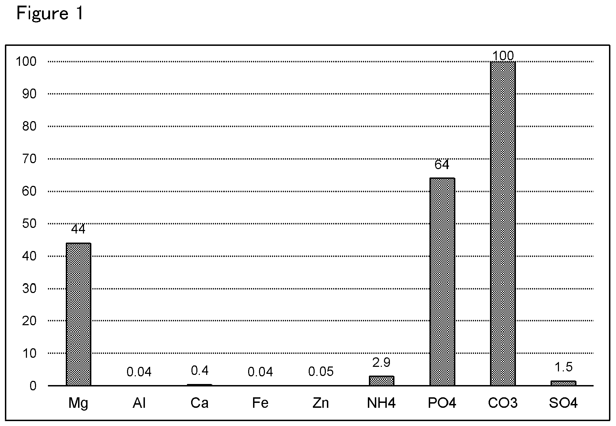

The amounts of the ions that constituted the precipitate in the second treated liquid of the above-described Comparative example 2 were measured by the methods shown in Table 5. As the ICP-MS spectrometer, "7900" manufactured by Agilent Technologies was used. As the ion chromatographer, "Integrion" manufactured by Thermo Fisher Scientific was used. The relative values to the weight of $CO_3^{2-}$ in the precipitate as 100 are shown in Figure 1.

Table 5

| Analysis method | Analyzed ion |
|---|---|
| ICP-MS | $Mg^{2+}$, $Al^{3+}$, $Ca^{2+}$, $Fe^{2+}$, $Zn^{2+}$ |
| Ion chromatography | $NH_4^+$, $PO_4^{3-}$, $CO_3^{2-}$, $SO_4^{2-}$ |

Since the main ions in the solid precipitate in the culture fluid after the treatment by basic magnesium carbonate are $Mg^{2+}$, $NH_4^+$, $PO_4^{3-}$, $CO_3^{2-}$ and $SO_4^{2-}$ as the result shown in Figure 1, the precipitation of a solid may be suppressed by reducing a part of or all of the ions.

(6) Measurement of amount of ion in culture fluid and treated liquid

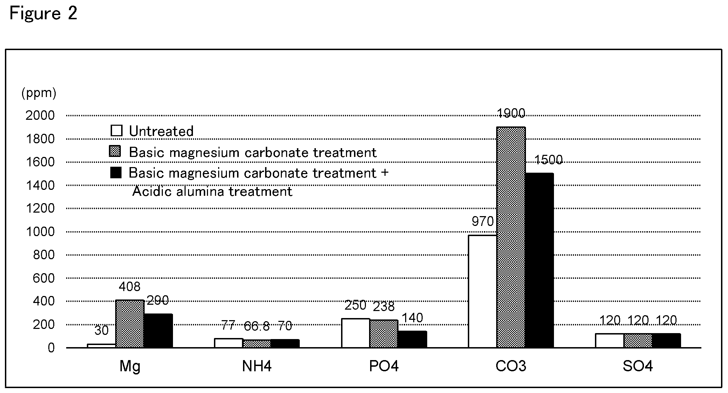

The amounts of the main ions specified by the above-described Example 3(5) were measured by a similar method to the above-described Example 3(5) among the ions contained in the untreated culture fluid (Comparative example 3), the first treated liquid treated with basic magnesium carbonate only (Comparative example 4) and the second treated liquid treated with acidic alumina in addition to basic magnesium carbonate (Example 3). The result is shown in Figure 2.

The concentrations of $Mg^{2+}$ and $CO_3^{2-}$ are increased by treating the untreated culture fluid with basic magnesium carbonate but $Mg^{2+}$, $PO_4^{3-}$ and $CO_3^{2-}$ are reduced by the subsequent acidic alumina treatment as the result shown in Figure 2. It was thus demonstrated by the result that the acidic alumina treatment can suppress precipitation by reducing $Mg^{2+}$, $PO_4^{3-}$ and $CO_3^{2-}$ as the main components of the precipitated solid.

Example 4: Effect to suppress precipitation by aluminum compound - example of the second present invention

Various aluminum compounds manufactured by Merck were used as the second adsorbent in the same condition as the above-described Example 1, and the amount of the precipitated solid was measured by eye for evaluation by the following standards. The result is shown in Table 6.

Excellent: No precipitation
Good: Precipitation amount corresponds to about 25% of the case of no second adsorbent
Average: Precipitation amount corresponds to about 50% of the case of no second adsorbent
Bad: Precipitation amount is similar to that of the case of no second adsorbent

Table 6

| 1st Adsorbent | 2nd Adsorbent | Solid precipitation amount |
| --- | --- | --- |
| - | - | Excellent |
| Basic magnesium carbonate | - | Bad |
| | Acidic alumina | Excellent |
| | Neutral alumina | Excellent |
| | Basic alumina | Good |
| | Aluminum hydroxide | Average |

Neutral alumina, basic alumina and aluminum hydroxide also have the effect to suppress precipitation in addition to acidic alumina as the result shown in Table 6. The reason why acidic alumina and neutral alumina have relatively higher effect to suppress precipitation than those of basic alumina and aluminum hydroxide may be that $PO_4^{3-}$ and $CO_3^{2-}$ as an anion causing precipitation are adsorbed thereon more effectively due to low negative charge density on the surface.

Example 5: Examination of treatment condition by acidic alumina - example of the second present invention

The effects on the suppression of solid precipitation in the case where the order of basic magnesium carbonate treatment and acidic alumina treatment was changed were compared on the basis of a similar method as Example 3. The result is shown in Table 7.

Table 7

| 1st Adsorbent | 2nd Adsorbent | Solid precipitation amount |
| --- | --- | --- |
| - | - | Excellent |
| Basic magnesium carbonate | - | Bad |
| Basic magnesium carbonate | Acidic alumina | Excellent |
| Acidic alumina | Basic magnesium carbonate | Excellent |
| Basic magnesium carbonate + Acidic alumina | | Good |

Even when the order of basic magnesium carbonate treatment and acidic alumina treatment was changed, the effects to suppress solid precipitation were on a level as the result shown in Table 7. The reason therefor may be that even when the culture fluid was first treated with an aluminum compound, $Mg^{2+}$, $PO_4^{3-}$ and $CO_3^{2-}$ contained in the culture fluid can be reduced. But when a mixture of basic magnesium carbonate and acidic alumina was used for the treatment, the effect to suppress precipitation was slightly decreased. The reason therefor may be possibly that acidic alumina directly adsorbs $Mg^{2+}$ and $CO_3^{2-}$ on the surface of basic magnesium carbonate and thus the adsorption capacity of acidic alumina is decreased.

Example 6: Effect to suppress precipitation in PBS solution of DNA - example of the second present invention

A 100 mg/L PBS solution of DNA derived from salmon sperm was prepared as a model solution. Then, the solution was treated in the condition shown in Table 8 similarly to the above-described Example 3, and then the DNA concentration was determined by measuring the absorbance at UV 260 nm. The result is shown in Table 8.

Table 8

| 1st Adsorbent | 2nd Adsorbent | DNA concentration | Solid precipitation amount |
|---|---|---|---|
| - | - | 100 mg/mL | Excellent |
| Basic magnesium carbonate | - | 52 mg/mL | Bad |
| Basic magnesium carbonate | Acidic alumina | 52 mg/mL | Excellent |

Even when a model solution containing a phosphate buffer solution as a solvent was treated, not only DNA as an impurity could be removed but also precipitation of a solid could be suppressed as the result shown in Table 8. The reason therefor may be that a phosphate ion as the main component of PBS can be adsorbed to be removed by acidic alumina.

Example 7: Effect to suppress precipitation by column filled with basic magnesium carbonate and acidic alumina - example of the second present invention

Column A was prepared by filling a commercially available column having a column volume (CV) of 1 mL with 1000 mg of basic magnesium carbonate. Column B was prepared by filling the same column with 500 mg of basic magnesium carbonate upstream and 500 mg of acidic alumina downstream by two layers. Column C of which layer order was opposite to Column B was prepared by filling the same column with 500 mg of acidic alumina upstream and 500 mg of basic magnesium carbonate downstream by two layers. The above-described IgG-containing CHO culture fluid (50 mL) was supplied into the Column A, Column B or Column C at a rate of 1 mL/min, and the eluate was obtained in increments of 10 CV. The degree of solid precipitation in the eluate was visually evaluated. The result is shown in Table 9.

Table 9

| | 1-10 CV | 11-20 CV | 21-30 CV | 31-40 CV | 41-50 CV |
|---|---|---|---|---|---|
| Column A | Bad | Bad | Bad | Bad | Bad |
| Column B | Excellent | Excellent | Excellent | Good | Good |
| Column C | Excellent | Excellent | Excellent | Good | Good |

A solid was precipitated in all of the fractions in the case of Column A that was filled with basic magnesium carbonate only as the result shown in Table 9.

On the one hand, the precipitation of a solid could be suppressed until 30 CV of supplied liquid amount and a precipitated solid amount could be also clearly suppressed after 31 CV in the cases of Column B and Column C in which a half of the column volume was replaced with acidic alumina in comparison with the case of Column A.

Example 8: Effect to suppress precipitation on various culture fluids - example of the second present invention

The culture fluids of various hosts to express various useful proteins were treated, and the degree of solid precipitation was visually evaluated similarly to the above-described Example 3. In addition, the recovery rate of the expressed protein was calculated on the basis of the densitometry intensity ratio of the band in general SDS-PAGE.

The result is shown in Table 10.

Table 10

| Host cell | Expressed protein | Basic magnesium carbonate treatment | Acidic alumina treatment | Expressed protein recovery rate | Solid precipitation amount |
|---|---|---|---|---|---|
| CHO | IgG | - | - | 1000 | Excellent |
| | | + | - | 99% | Bad |
| | | + | + | 99% | Excellent |
| E. coli | VHH | - | - | 100% | Excellent |
| | | + | - | 92% | Bad |
| | | + | + | 76% | Excellent |
| Pichia veast | scFv | - | - | 100% | Excellent |
| | | + | - | 93% | Bad |
| | | + | + | 94% | Excellent |

A solid was precipitated by the treatment of basic magnesium carbonate only but the precipitation of a solid could be suppressed by the additional acidic alumina treatment in all of the cases as the result shown in Table 10. The expressed protein recovery rate of VHH was decreased to 76% but 90% or more of the expressed protein recovery rate of IgG and scFv were maintained.

**Claims**

1. A method for purifying a useful substance from a useful substance-containing liquid, the method comprising the steps of:

   contacting the useful substance-containing liquid with a hardly-soluble magnesium compound,
   removing the hardly-soluble magnesium compound from the useful substance-containing liquid contacted with the hardly-soluble magnesium compound to obtain a treated liquid, and
   adjusting pH of the treated liquid to 8.0 or less.

2. The method according to claim 1, wherein pH of the useful substance-containing liquid is more than 8.0.

3. The method according to claim 1 or 2, wherein the pH of the treated liquid is adjusted to 5.0 or more and 8.0 or less.

4. The method according to any one of claims 1 to 3, wherein the useful substance-containing liquid is a cell culture fluid or a partially purified cell culture fluid.

5. The method according to any one of claims 1 to 4, wherein the hardly-soluble magnesium compound is one or more selected from magnesium carbonate, magnesium hydroxide and magnesium oxide.

6. The method according to any one of claims 1 to 5, wherein the hardly-soluble magnesium compound comprises magnesium carbonate.

7. The method according to any one of claims 1 to 6, wherein the useful substance-containing liquid comprises nucleic acid, and the useful substance-containing liquid is contacted with the hardly-soluble magnesium compound to adsorb at least a part of the nucleic acid on the hardly-soluble magnesium compound.

8. The method according to any one of claims 1 to 7, wherein the useful substance-containing liquid comprises an antibody or an antibody-like molecule as the useful substance.

9. The method according to any one of claims 1 to 7, wherein the useful substance-containing liquid comprises a virus or a virus-like particle as the useful substance.

10. A method for purifying a useful substance from a useful substance-containing liquid, the method comprising the steps of:

contacting the useful substance-containing liquid with a hardly-soluble magnesium compound, and
contacting the useful substance-containing liquid with an aluminum compound.

11. The method according to claim 10, wherein the useful substance-containing liquid is contacted with the aluminum compound after the useful substance-containing liquid is contacted with the hardly-soluble magnesium compound.

12. The method according to claim 10, wherein the useful substance-containing liquid is contacted with the hardly-soluble magnesium compound after the useful substance-containing liquid is contacted with the aluminum compound.

13. The method according to any one of claims 10 to 12, wherein the useful substance-containing liquid is a cell culture fluid or a partially purified cell culture fluid.

14. The method according to any one of claims 10 to 13, wherein one or more ions selected from a magnesium ion, a phosphate ion and a carbonate ion are adsorbed on the aluminum compound.

15. The method according to any one of claims 10 to 14, wherein the hardly-soluble magnesium compound is one or more selected from magnesium carbonate, magnesium hydroxide and magnesium oxide.

16. The method according to any one of claims 10 to 15, wherein the hardly-soluble magnesium compound comprises magnesium carbonate.

17. The method according to any one of claims 10 to 16, wherein the aluminum compound is one or more selected from aluminum oxide and aluminum hydroxide.

18. The method according to any one of claims 10 to 17, wherein the useful substance-containing liquid comprises an antibody or an antibody-like molecule as the useful substance.

19. The method according to any one of claims 10 to 18, wherein the useful substance-containing liquid comprises a virus or a virus-like particle as the useful substance.

Figure 1

Figure 2

<table>
<tr><td colspan="2" style="text-align:center">INTERNATIONAL SEARCH REPORT</td><td colspan="2">International application No.<br>**PCT/JP2021/028824**</td></tr>
</table>

**A. CLASSIFICATION OF SUBJECT MATTER**

*C12P 21/08*(2006.01)i; *B01J 20/04*(2006.01)i; *C07K 1/14*(2006.01)i; *C07K 14/005*(2006.01)i; *C07K 16/00*(2006.01)i;
*C12N 7/02*(2006.01)i
FI:  C12P21/08; C12N7/02; B01J20/04 A; B01J20/04 B; C07K16/00; C07K1/14; C07K14/005

According to International Patent Classification (IPC) or to both national classification and IPC

**B. FIELDS SEARCHED**

Minimum documentation searched (classification system followed by classification symbols)

C12P1/00-41/00; C07K1/00-19/00; C12N1/00-7/08

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN); PubMed

**C. DOCUMENTS CONSIDERED TO BE RELEVANT**

| Category* | Citation of document, with indication, where appropriate, of the relevant passages | Relevant to claim No. |
|---|---|---|
| Y | WO 2020/045290 A1 (KANEKA CORPORATION) 05 March 2020 (2020-03-05)<br>    claims 1, 2, 4 and 5, paragraphs [0002], [0009], examples | 1-9 |
| Y | WO 2018/203541 A1 (KANEKA CORPORATION) 08 November 2018 (2018-11-08)<br>    paragraph [0093] | 1-9 |
| Y | WO 2018/021012 A1 (KANEKA CORPORATION) 01 February 2018 (2018-02-01)<br>    paragraphs [0052]-[0053] | 1-9 |
| Y | JP 2010-530734 A (LGOCYTE PHARMACEUTICALS, INC.) 16 September 2010<br>(2010-09-16)<br>    claims | 9 |
| Y | JP 2000-262280 A (ASAHI OPTICAL CO., LTD.) 26 September 2000 (2000-09-26)<br>    claims | 9 |
| Y | JP 2017-055766 A (PHILIP MORRIS PRODUCTS S.A.) 23 March 2017 (2017-03-23)<br>    claims | 9 |

☑ Further documents are listed in the continuation of Box C.      ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **06 October 2021** | **26 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2021/028824** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2009-508486 A (WYETH LLC) 05 March 2009 (2009-03-05) entire text, in particular, claims | 1-9 |

Form PCT/ISA/210 (second sheet) (January 2015)

## INTERNATIONAL SEARCH REPORT

International application No.

**PCT/JP2021/028824**

**Box No. III    Observations where unity of invention is lacking (Continuation of item 3 of first sheet)**

This International Searching Authority found multiple inventions in this international application, as follows:

The invention as in claims 1-9 and the invention as in claims 10-19 share the feature of "a method for refining a useful substance from a useful substance-containing liquid," the method including "a step for bringing the useful substance-containing liquid into contact with a poorly soluble magnesium compound." However, this feature does not make a contribution over the prior art in light of the content disclosed in document 1, and thus this feature cannot be said to be a special technical feature. Additionally, there is no other identical or corresponding special technical feature between these inventions.

Moreover, the claims are classified into the two inventions having the special technical features described below.

(Invention 1) Claims 1-9

The invention that pertains to a method for refining a useful substance from a useful substance-containing liquid, the method being characterized by including: a step for bringing the useful substance-containing liquid into contact with a poorly soluble magnesium compound; a step for obtaining a processed liquid by removing the poorly soluble magnesium compound from the useful substance-containing liquid that have been brought into contact with the poorly soluble magnesium compound; and a step for adjusting the pH of the processed liquid to 8.0 or lower.

(Invention 2) Claims 10-19

The invention that pertains to a method for refining a useful substance from a useful substance-containing liquid, the method being characterized by including: a step for bringing the useful substance-containing liquid into contact with a poorly soluble magnesium compound; and a step for bringing the useful substance-containing liquid into contact with an aluminum compound.

Claims 10-19 are not inventions of the same category that includes all invention-defining features of claim 1. In addition, completion of examination on the claims classified as invention 1 does not substantially eliminate the need for additional prior art search and determination for examination on claims 10-19. Moreover, it cannot justifiably be said that examining claims 10-19 together with claims 1-9 would be efficient. As such, claims 10-19 cannot be classified as invention 1.

Document 1: WO 2020/045290 A1 (KANEKA CORPORATION) 05 March 2020 (2020-03-05), claims 1, 2, 4 and 5, paragraphs [0002], [0009], examples (Family: none)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-9**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

INTERNATIONAL SEARCH REPORT
Information on patent family members

International application No.

**PCT/JP2021/028824**

| Patent document cited in search report | | | Publication date (day/month/year) | Patent family member(s) | | | Publication date (day/month/year) |
|---|---|---|---|---|---|---|---|
| WO | 2020/045290 | A1 | 05 March 2020 | (Family: none) | | | |
| WO | 2018/203541 | A1 | 08 November 2018 | (Family: none) | | | |
| WO | 2018/021012 | A1 | 01 February 2018 | US | 2019/0153072 | A1 | |
| | | | | paragraphs [0073]-[0074] | | | |
| | | | | EP | 3492493 | A1 | |
| JP | 2010-530734 | A | 16 September 2010 | US | 2010/0150961 | A1 | |
| | | | | WO | 2008/113011 | A2 | |
| | | | | claims | | | |
| | | | | EP | 2134360 | A2 | |
| | | | | KR | 10-2010-0015562 | A | |
| JP | 2000-262280 | A | 26 September 2000 | GB | 2348886 | A | |
| | | | | claims | | | |
| | | | | DE | 10013017 | A1 | |
| | | | | FR | 2791698 | A1 | |
| JP | 2017-055766 | A | 23 March 2017 | US | 2013/0317197 | A1 | |
| | | | | WO | 2012/055986 | A1 | |
| | | | | claims | | | |
| | | | | EP | 2632562 | A1 | |
| | | | | CN | 103228327 | A | |
| | | | | KR | 10-2013-0122947 | A | |
| JP | 2009-508486 | A | 05 March 2009 | US | 2007/0066806 | A1 | |
| | | | | WO | 2007/035283 | A1 | |
| | | | | entire text, in particular, claims | | | |
| | | | | EP | 1934242 | A1 | |
| | | | | CN | 101263155 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015111606 A
- JP 2017055766 A
- WO 2020045290 A
- JP 2008137827 A
- JP 2020144757 A
- JP 2020144758 A